# EUROPÄISCHE PATENTSCHRIFT

(11) **EP 1 719 519 B1**
(45) Veröffentlichungstag und Bekanntmachung des Hinweises auf die Patenterteilung: **24.10.2007**
(21) Anmeldenummer: 05004035.1
(22) Anmeldetag: 24.02.2005
(51) Int. Cl.: A61K 36/67, A23L 1/30, A61K 33/06, A61K 33/34, A61K 31/59

(54) **Nahrungsergänzungsmittelpräparat-Set**
Dietary supplement kit
Complément alimentaire sous forme de kit

(43) Veröffentlichungstag der Anmeldung: 08.11.2006
(60) Teilanmeldung: 06011271.1
(73) Patentinhaber: PM-International AG, 1618 Luxembourg (LU)
(72) Erfinder: Schmitt, Gerhard Dr., 64625 Bensheim (DE)
(74) Vertreter: Zech, Stefan Markus

(56) Entgegenhaltungen:
- US-A- 5 514 382
- US-A- 5 536 506
- US-A- 6 048 846
- US-A1- 2004 052 873
- US-A1- 2004 191 388
- US-B1- 6 440 450
- US-B1- 6 572 899

## Beschreibung

*Die Erfindung betrifft ein Nabrungsergänzungsmittelpräparat-Set umfassend als Grundsubstanz und als Additiv je ein Nahrungsergänzungsmittelpräparat enthaltend die Mineralstoffe und Spurenelemente (Wirkstoffe) Vitamin D, Calcium, Magnesium, Selen, Kupfer, Zink und Chrom.*

Magnesium beeinflusst die Reizleitungsvorgänge im zentralen und peripheren Nervensystem.

Selen ist eine antioxidative Schutzsubstanz. Es ist Baustein in vielen Enzymsystemen.

Chrom unterstützt den Glucose-Toleranzfaktor und erhöht damit das Ansprechverhalten auf Insulin. Es erfüllt somit eine wichtige Funktion im Zuckerstoffwechsel.

*Eisen ist ein Additiv, das sich unter anderem bei starker Übersäuerung als vorteilhaft erwiesen hat. Besonders Hochleistungssportler und Frauen sollten daher auf ein Nahrungsergänzungsmittelpräparat mit Eisenanteil zurückgreifen, um die Hämoglobinsynthese sicher Zu stellen.*

*In* US-A-5 536 506 *werden eine Mischung und ein Verfahren zu ihrer Erstellung zur Verbesserung der Magen-Darm-Aufnahme und der systematischen Verwendung von Nahrungsstoffen bzw. Nahrungsergänzungsstoffen offenbart. Die Mischung weist dabei neben Piperin eine Vielzahl von unterschiedlichen Mineralstoffen und Spurenelementen auf.*

US-A-6 048 846 *beschreibt eine Zusammensetzung zur Bebandlung des menschlichen Körpers, die eine Kombination von mindestens einem Hormon, mindestens einer Aminosäure, mindestens einem Enzym und*/*oder Vitamin und mindestens einem Mineralstoff umfasst. Dabei sind die Relationsverhältnisse des Hormons, der Aminosäure, des Enzyms und*/*oder Vitamins und des Mineralstoffs so aufeinander abgestimmt, dass diese Stoffe in optimalen Mengen im Körper gespeichert werden können*.

*In* US 200470191388 A1 *wird eine Zusammensetzung dargelegt, die als ein hydratisierendes Getränk verwendbar ist, die mindestens ein Carbonhydrat-Komplex, mindestens ein Chelat-Elektrolyt, Betain und Piperin, aufweist.*

US-B1-6 572 899 *erläutert eine Zusammensetzung die eine wirkungsvolle Kombination von Nährstoffen enthält, die im Hinblick auf die Bekämpfung von Gedächtnisverlust, Demenzen und der Alzheimer-Krankheit zusammengestellt worden sind.*

US-A-5 514 382 *offenbart eine tägliche Vitamin- und Mineralstoffergänzung für Frauen, die unterschiedliche Vitamine, Spurenelemente und Mineralstoffe umfasst, wobei eine erste Variante dieser Ergänzung speziell für Frauen bis zum vierzigsten Lebensjahr und eine zweite Variante dieser Ergänzung speziell für Frauen ab dem vierzigsten Lebensjahr zusammengestellt worden ist*.

Nachteilig bei bekannten Nahrungsergänzungsmittelpräparaten ist die hohe Zeitdauer bis zur Resorption (Aufnahme) der Mineralstoffe und Spurenelemente nach der Einnahme. Ferner können sich aufgrund ungenügender Abstimmung die enthaltenen Mineralstoffe und Spurenelemente gegenseitig behindern. Beispielsweise können die Resorptionskanäle für einen Mineralstoff bzw. ein Spurenelement durch ein oder mehrere andere Mineralstoffe bzw. Spurenelemente besetzt sein. Auch kann der Transport der einzelnen Wirkstoffe an die Wirkstätte durch andere Wirkstoffe behindert werden.

Hinzu kommt, dass mehrere Wirkstoffe untereinander nicht kompatibel sein können. Beispielsweise behindern sich Calcium und Magnesium gegenseitig, wenn ihr Mengenverhältnis und/oder ihre molekulare Einbindung nicht stimmen. Falsche Mengenverhältnisse und/oder molekulare Einbindung können beispielsweise zur Komplexbildung führen.

Aufgabe der vorliegenden Erfindung ist somit, ein *Nahrungsergänzungsmittelpräparat-Set* mit verbesserten Resorptionseigenschaften für Mineralstoffe und Spurenelemente anzugeben, *wobei Grundsubstanz und*/*oder Additiv für die eigenständige Verwendung geeignet sind und wobei Grundsubstanz und Additiv zur Einstellung eines gewünschten Eisenanteils in beliebigem Verhältnis mischbar sind.* Ferner sollen die verschiedenen Mineralstoffe und Spurenelemente derart aufeinander abgestimmt sein, dass sie sich nicht gegenseitig in ihrer Resorption und in ihrem Transport an die Wirkungsstätte stören.

*Nach einem Kerngedanke der vorliegenden Erfindung umfasst das Nahmngsergänzungsmittelpräparat-Set als Grundsubstanz ein erstes Nahrungsergänzungsmittelpräparat nach der Erfindung, das kein Eisen enthält, und als Additiv ein zweites Nahrungsergänzungsmittelpräparat nach der Erfindung, das Eisen enthält. Die Grundsubstanz und*/*oder das Additiv können dabei für die eigenständige Verwendung geeignet sein. Insbesondere sind Grundsubstanz und Additiv zur Einstellung eines gewünschten Eisenanteils in beliebigem Verhältnis mischbar. Die Bedeutung von Eisen für bestimmte Menschengruppen, insbesondere Sportler und*/*oder Frauen, wurde bereits obenstehend dargelegt. Das Nahrungsergänzungsmitelpräparat-Set ermöglicht es dem jeweiligen Verwender, den Eisenanteil entsprechend seinen individuellen Bedürfnissen durch entsprechendes Mischen von Grundsubstanz und additiv einzustellen, so dass ihm dadurch ein optimal zusammengesetztes Präparat zur Verfügung steht.*

Diese Aufgabe wird gelöst durch ein *Nahrungsergänzungsmittelpräparat-Set* mit den Merkmalen nach Anspruch 1. Vorteilhafte Weiterbildungen ergeben sich aus den abhängigen Ansprüchen.

Gemäß Anspruch 1 *enthalten die Nahrungsergänzungsmittelpräparate im Nahrungsergänzungsmittelpräparat-Set jeweils* neben den Mineralstoffen und Spurenelementen Vitamin D, Calcium, Magnesium, Selen, Kupfer, Zink und Chrom mindestens einen Katalysator und/oder Wirkstoff (auch als REC - Resorption Enhancing Catalyst bezeichnet), vorzugsweise in Pulverform, der die Resorption der Mineralstoffe und/oder Spurenelemente steigert und/oder verbessert und/oder beschleunigt.

Die Vorteile der Erfindung liegen insbesondere in den verbesserten Resorptionseigenschaften (einschließlich des verbesserten Transports der Wirkstoffe an ihre Wirkstätte) *der Nahrungsergänzungsmittelpräparate.* Dadurch werden die Mineralstoffe und/oder Spurenelemente schneller in den Körper aufgenommen und können mit geringerem Zeitabstand zur Einnahme ihre Wirkungen entfalten. Auch kann der Katalysator und/oder Wirkstoff die Wirksamkeit der Mineralstoffe und/oder Spurenelemente im Körper verbessern. *Die Nahrungsergänzungsmittelpräparate eignen* sich insbesondere dazu, Osteoporose und Übersäuerung entgegenzuwirken.

Nach einer vorteilhaften Weiterbildung umfasst der Katalysator und/oder Wirkstoff ein Schwarzpfefferextrakt bzw. er besteht daraus. Aus Schwarzem Pfeffer (piper nigrum) als Ausgangssubstanz lässt sich ein besonders effektiver Katalysator und/oder Wirkstoff gewinnen.

Gemäß einer bevorzugten Ausführungsform umfasst der Katalysator und/oder Wirkstoff Piperin (eine chemische Variante ist 1-Piperoylpiperidin C₁₇H₁₉NO₃), besonders wirkungsvoll als Alkaloid, bzw. er besteht daraus. Aus Schwarzem Pfeffer (piper nigrum) als Ausgangssubstanz lässt sich ein Piperin gewinnen, das als Katalysator und/oder Wirkstoff besonders effektiv ist.

Von Vorteil ist, wenn der Piperinanteil im Katalysator und/oder Wirkstoff im Bereich von 90 Prozent bis 100 Prozent liegt, insbesondere im Bereich von 92 bis 100 Prozent, vorzugsweise bei mindestens 95 Prozent.

Eine Weiterbildung sieht vor, dass der Katalysator und/oder Wirkstoff vollständig oder zumindest weitestgehend in seiner molekularen Struktur, wie sie im Schwarzen Pfeffer vorliegt, belassen bleibt. Es soll sich dabei insbesondere um ein aus der Pflanze Schwarzer Pfeffer gewonnene Substanz und nicht um ein künstlich chemisch erzeugtes Produkt handeln. Eine spezielle Vermahlung kann die (zumindest weitestgehende) Aufrechterhaltung der natürlichen molekularen Struktur gewährleisten.

Als besonderes vorteilhaft hat es sich erwiesen, wenn *das erste und*/*oder zweite Nahrungsergänzungsmittelpräparat des Nahrungsergänzungsmittelpräparat-Sets* Magnesiumhydroxidcarbonat enthält. Viele der im Markt angebotenen Magnesiumverbindungen sind im allgemeinen schwer resorbierbar. Üblich ist bisher vor allem die Verwendung von Magnesiumcarbonat. Demgegenüber führt die Verwendung von Magnesiumhydroxidcarbonat zu einer verbesserten Resorbierbarkeit des Magnesium sowie zu einer Steigerung von dessen Wirksamkeit.

Zweckmäßigerweise enthält *das erste und*/*oder zweite Nahrungsergänzungsmittelpräparat des Nahrungsergänzungsmittelpräparat-Sets* je nach Ausführungsvariante Beta-Carotin (Provitamin A) und/oder Kalium und/oder Natrium.

Eine bevorzugte Weiterbildung des *Nahrungsergänzungsmittelpräparat-Set* sieht vor, dass *bei dem ersten und*/*oder dem zweiten Nahrungsergänzungsmittelpräparat jeweils*
der Kaliumanteil im Präparat zwischen 0,15 und 35 Gew.% liegt, vorzugsweise zwischen 0,5 und 10 Gew.%, insbesondere bei 0,8 Gew.%, und/oder
der Calciumanteil im Präparat zwischen 1,6 und 17 Gew.% liegt, vorzugsweise zwischen 2,5 und 10 Gew.%, insbesondere bei 4,5 Gew.% und/oder
der Magnesiumanteil im Präparat zwischen 0,8 und 5,0 Gew.% liegt, vorzugsweise zwischen 2 und 3 Gew.%, insbesondere bei 2,2 Gew.%, und/oder
der Zinkanteil im Präparat zwischen 0,015 und 0,2 Gew.% liegt, vorzugsweise zwischen 0,05 und 0,1 Gew.%, insbesondere bei 0,08 Gew.%, und/oder
der Kupferanteil im Präparat zwischen 0,0015 und 0,017 Gew.% liegt, vorzugsweise zwischen 0,005 und 0,015 Gew.%, insbesondere bei 0,011 Gew.%, und/oder
der Chromanteil im Präparat zwischen 0,00015 und 0,0017 Gew.% liegt, vorzugsweise zwischen 0,0002 und 0,0008 Gew.%, insbesondere bei 0,00028 Gew.%, und/oder der Selenanteil im Präparat zwischen 0,00015 und 0,0012 Gew.% liegt, vorzugsweise zwischen 0,0002 und 0,0006 Gew.%, insbesondere bei 0,00028 Gew.%, und/oder
der Eisenanteil im Präparat bei nahezu 0 Gew.% oder zwischen 0,015 und 0,09 Gew.% liegt, vorzugsweise zwischen 0,03 und 0,09 Gew.%, insbesondere bei 0,08 Gew.%, und/oder
der Vitamin-D-Anteil im Präparat zwischen 0,00001 und 0,00008 Gew.%, liegt, vorzugsweise zwischen 0,00002 und 0,00006 Gew.%, insbesondere bei 0,00004 Gew.%, und/oder
der Anteil des Katalysators und/oder Wirkstoffs zur Resorptionsverbesserung, insbesondere der Piperinanteil, im Präparat zwischen 0,15 und 1,7 Gew.% liegt, vorzugsweise zwischen 0,4 und 1,2 Gew.%, insbesondere bei 0,8 Gew.%.

Zweckmäßige und bevorzugte Ausführungsvarianten sehen vor, dass *bei dem Nahrungsergänzungsmittelpräparat-Set das erste und*/*oder zweite Nahrungsergänzungsmittelpräparat* in Pulverform vorliegt und/oder ausgebildet und bestimmt ist zur Herstellung einer wässrigen Lösung und/oder ausgebildet und bestimmt ist zur Herstellung eines Getränks, insbesondere eines Mineral-Basen-Gertränks. Als Getränk lässt sich *erste und*/*oder zweite Präparat des Präparat-Sets* besonders einfach, flexibel und schnell einnehmen, wodurch eine schnelle Wirkung sichergestellt wird. Auch ermöglicht ein Getränk die problemlose und bequeme regelmäßige Verwendung, ohne die sich die gewünschten Wirkungen beim Menschen in der Regel nicht erzielen lassen.

Nachfolgend werden die charakteristischen Werte eines Ausführungsbeispiels des *Nahrungsergängsmittelpräparats-Sets* gemäß der Erfindung angeführt.

Ausgehend von einer Nahrungsergänzungsmittelpräparat-Portion von 6 g (Gramm), was der einzunehmenden Tagesmenge entspricht, gibt folgende Tabelle typische Mengenwerte der einzelnen Mineralstoffe bzw. Spurenelemente innerhalb dieser Portion an. Das heißt die Tagesportion von 6 g enthält 50 mg (Milligramm) Kalium, 270 mg Calcium, usw. Weitere verwendete Einheit: µg (Mikrogramm). Ferner sind die Grenzen angegeben, innerhalb derer die jeweilige Substanz variiert werden kann. Neben den angegebenen Substanzen sind in der Portion noch weitere Substanzen enthalten, die beispielsweise der Aromatisierung und/oder der Löslichkeit des Nahrungsergänzungsmittelpräparats dienen. Dadurch wird eine guten Löslichkeit und eine geschmackliche Akzeptanz des Nahrungsergänzungsmittelpräparats erreicht.

| Mineralstoff bzw. Spurenelement | Menge in einer 6-Gramm-Portion | Grenzwerte |
|---|---|---|
| Kalium | 50 mg | 10 mg - 2.000 mg |
| Calcium | 270 mg | 100 mg - 1.000 mg |
| Magnesium | 134 mg | 50 mg - 300 mg |
| Zink | 5 mg | 1 mg - 11 mg |
| Kupfer | 670 µg | 100 µg - 1 mg |
| Chrom | 17 µg | 10 µg - 100 µg |
| Selen | 17 µg | 10 µg - 70 µg |
| Eisen | 5 mg | 1 mg - 5 mg |
| Vitamin D | 2,5 µg | 1 µg - 5 µg |

Ferner kann die Menge an Piperin pro 6-Gramm-Portion typischerweise bei 0,1 mg bis 10 mg, vorzugsweise bei 0,3 mg bis 2 mg, insbesondere bei 0,5 mg liegen.

Im Nahrungsergänzungsmittelpräparat-Set entspricht die Grundsubstanz der obigen Zusammensetzung bis auf den Eisenwert, der bei 0 Gramm liegt. Das Additiv entspricht hingegen der vorstehenden Zusammensetzung einschließlich des Eisenwertes. Den individuell gewünschten Eisenwert kann der Verwender dann durch entsprechendes Mischen von Grundsubstanz und Additiv einstellen.

## Patentansprüche

1. Nahrungsergänzungsmittelpräparat-Set umfassend als Grundsubstanz ein erstes Nahrungsergänzungsmittelpräparat enthaltend die Mineralstoffe und Spurenelemente Vitamin D, Calcium, Magnesium, Selen, Kupfer, Zink und Chrom und mindestens einen Katalysator und/oder Wirkstoff, der die Resorption der Mineralstoffe und/oder Spurenelemente steigert und/oder verbessert und/oder beschleunigt, wobei das erste Präparat kein Eisen enthält, und
als Additiv ein zweites Nahrungsergänzungsmittelpräparat enthaltend die Mineralstoffe und Spurenelemente Vitamin D, Calcium, Magnesium, Selen, Kupfer, Zink und Chrom und mindestens einen Katalysator und/oder Wirkstoff, der die Resorption der Mineralstoffe und/oder Spurenelemente steigert und/oder verbessert und/oder beschleunigt, wobei das zweite Präparat Eisen enthält,
wobei Grundsubstanz und/oder Additiv für die eigenständige Verwendung geeignet sind und
wobei Grundsubstanz und Additiv zur Einstellung eines gewünschten Eisenanteils in beliebigem Verhältnis mischbar sind.

2. Nahrungsergänzungsmittelpräparat-Set nach Anspruch 1,
**dadurch gekennzeichnet, dass**
bei dem ersten und/oder dem zweiten Präparat jeweils der zumindest eine Katalysator und/oder Wirkstoff in Pulverform vorliegt.

3. Nahrungsergänzungsmittelpräparat-Set nach Anspruch 1oder 2,
**dadurch gekennzeichnet, dass**
bei dem ersten und/oder dem zweiten Präparat jeweils der Katalysator und/oder Wirkstoff ein Schwarzpfefferextrakt umfasst und/oder daraus besteht.

4. Nahrungsergänzungsmittelpräparat-Set nach einem der vorhergehenden Ansprüche,
**dadurch gekennzeichnet, dass**
bei dem ersten und/oder dem zweiten Präparat jeweils der Katalysator und/oder Wirkstoff Piperin (chemische Bezeichnung 1-Piperoylpiperidin C₁₇H₁₉NO₃), insbesondere als Alkaloid, umfasst und/oder daraus besteht, wobei das Piperin vorzugsweise aus Schwarzem Pfeffer gewonnen ist.

5. Nahrungsergänzungsmittelpräparat-Set nach Anspruch 4,
**dadurch gekennzeichnet, dass**
bei dem ersten und/oder dem zweiten Präparat jeweils der Piperinanteil im Katalysator und/oder Wirkstoff im Bereich von 90 Gew.% bis 100 Gew.% liegt, insbesondere im Bereich von 92 bis 100 Gew.%, vorzugsweise bei mindestens 95 Gew.%.

6. Nahrungsergänzungsmittelpräparat-Set nach einem der vorhergehenden Ansprüche,
**dadurch gekennzeichnet, dass**
bei dem ersten und/oder dem zweiten Präparat jeweils der Katalysator und/oder Wirkstoff vollständig oder zumindest weitestgehend in seiner molekularen Struktur, wie sie im Schwarzen Pfeffer vorliegt, belassen bleibt.

7. Nahrungsergänzungsmittelpräparat-Set nach einem der vorhergehenden Ansprüche,
**dadurch gekennzeichnet, dass**
bei dem ersten und/oder dem zweiten Präparat jeweils das Präparat Magnesiumhydroxidcarbonat enthält.

8. Nahrungsergänzungsmittelpräparat-Set nach einem der vorhergehenden Ansprüche,
**dadurch gekennzeichnet, dass**
bei dem ersten und/oder dem zweiten Präparat jeweils das Präparat Beta-Carotin (Provitamin A) und/oder Kalium und/oder Natrium enthält.

9. Nahrungsergänzungsmittelpräparat-Set nach einem der vorhergehenden Ansprüche,
**dadurch gekennzeichnet, dass**
bei dem ersten und/oder dem zweiten Präparat jeweils
der Kaliumanteil im Präparat zwischen 0,15 und 35 Gew.% liegt, vorzugsweise zwischen 0,5 und 10 Gew.%, insbesondere bei 0,8 Gew.%, und/oder
der Calciumanteil im Präparat zwischen 1,6 und 17 Gew.% liegt, vorzugsweise zwischen 2,5 und 10 Gew.%, insbesondere bei 4,5 Gew.% und/oder
der Magnesiumanteil im Präparat zwischen 0,8 und 5,0 Gew.% liegt, vorzugsweise zwischen 2 und 3 Gew.%, insbesondere bei 2,2 Gew.%, und/oder
der Zinkanteil im Präparat zwischen 0,015 und 0,2 Gew.% liegt, vorzugsweise zwischen 0,05 und 0,1 Gew.%, insbesondere bei 0,08 Gew.%, und/oder
der Kupferanteil im Präparat zwischen 0,0015 und 0,017 Gew.% liegt, vorzugsweise zwischen 0,005 und 0,015 Gew.%, insbesondere bei 0,011 Gew.%, und/oder
der Chromanteil im Präparat zwischen 0,00015 und 0,0017 Gew.% liegt, vorzugsweise zwischen 0,0002 und 0,0008 Gew.%, insbesondere bei 0,00028 Gew.%, und/oder
der Selenanteil im Präparat zwischen 0,00015 und 0,0012 Gew.% liegt, vorzugsweise zwischen 0,0002 und 0,0006 Gew.%, insbesondere bei 0,00028 Gew.%, und/oder
der Eisenanteil im Präparat bei 0 Gew.% oder zwischen 0,015 und 0,09 Gew.% liegt, vorzugsweise zwischen 0,03 und 0,09 Gew.%, insbesondere bei 0,08 Gew.%, und/oder
der Vitamin-D-Anteil im Präparat zwischen 0,00001 und 0,00008 Gew.%, liegt, vorzugsweise zwischen 0,00002 und 0,00006 Gew.%, insbesondere bei 0,00004 Gew.%, und/oder
der Anteil des Katalysators und/oder Wirkstoffs zur Resorptionsverbesserung, insbesondere der Piperinanteil, im Präparat zwischen 0,0015 und 0,17 Gew.% liegt, vorzugsweise zwischen 0,005 und 0,034 Gew.%, insbesondere bei 0,008 Gew.%

10. Nahrungsergänzungsmittelpräparat-Set nach einem der vorhergehenden Ansprüche,
**dadurch gekennzeichnet, dass**
bei dem ersten und/oder dem zweiten Präparat jeweils das Präparat in Pulverform vorliegt und/oder ausgebildet und bestimmt ist zur Herstellung einer wässrigen Lösung und/oder ausgebildet und bestimmt ist zur Herstellung eines Getränks, insbesondere eines Mineral-Basen-Getränks.

## Claims

1. Nutritional supplement preparation set comprising as basic substance, a first nutritional supplement preparation containing the mineral materials and trace elements vitamin D, calcium, magnesium, selenium, copper, zinc and chromium and at least one catalyst and/or active ingredient, which increases and/or improves and/or accelerates the resorption of the mineral materials and/or trace elements, wherein the first preparation contains no iron, and as an additive a second nutritional supplement preparation containing the mineral materials and trace elements vitamin D, calcium, magnesium, selenium, copper, zinc and chromium and at least one catalyst and/or active ingredient, which increases and/or improves and/or accelerates the resorption of the mineral materials and/or trace elements, wherein the second preparation contains iron, wherein basic substance and/or additive are suitable for independent use and wherein basic substance and additive can be mixed in any ratio for adjustment of a required iron proportion.

2. Nutritional supplement preparation set according to claim 1, **characterised in that** in the first and/or the second preparation, in each case the at least one catalyst and/or active ingredient is present in powder form.

3. Nutritional supplement preparation set according to claim 1 or 2, **characterised in that** in the first and/or the second preparation, in each case the catalyst and/or active ingredient comprises a black pepper extract and/or consists thereof.

4. Nutritional supplement preparation set according to one of the preceding claims, **characterised in that** in the first and/or the second preparation, in each case the catalyst and/or active ingredient comprises piperin (chemical designation 1-piperoylpiperidine C₁₇H₁₉NO₃), in particular as alkaloid, and/or consists thereof, wherein the piperin is preferably recovered from black pepper.

5. Nutritional supplement preparation set according to claim 4, **characterised in that** in the first and/or the second preparation, in each case the piperin proportion in the catalyst and/or active ingredient lies in the range from 90 wt.% to 100 wt.%, in particular in the range from 92 to 100 wt.%, preferably at least at 95 wt.%.

6. Nutritional supplement preparation set according to one of the preceding claims, **characterised in that** in the first and/or the second preparation, in each case the catalyst and/or active ingredient completely or at least to the greatest possible extent remains left in its molecular structure, as exists in black pepper.

7. Nutritional supplement preparation set according to one of the preceding claims, **characterised in that** in the first and/or the second preparation, in each case the preparation contains magnesium hydroxide carbonate.

8. Nutritional supplement preparation set according to one of the preceding claims, **characterised in that** in the first and/or the second preparation, in each case the preparation contains beta-carotene (provitamin A) and/or potassium and/or sodium.

9. Nutritional supplement preparation set according to one of the preceding claims, **characterised in that** in the first and/or the second preparation, in each case the potassium proportion in the preparation lies between 0.15 and 35 wt.%, preferably between 0.5 and 10 wt.%, in particular at 0.8 wt.%, and/or the calcium proportion in the preparation lies between 1.6 and 17 wt.%, preferably between 2.5 and 10 wt.%, in particular at 4.5 wt.% and/or the magnesium proportion in the preparation lies between 0.8 and 5.0 wt.%, preferably between 2 and 3 wt.%, in particular at 2.2 wt.%, and/or the zinc proportion in the preparation lies between 0.015 and 0.2 wt.%, preferably between 0.05 and 0.1 wt.%, in particular at 0.08 wt.%, and/or the copper proportion in the preparation lies between 0.0015 and 0.017 wt.%, preferably between 0.005 and 0.015 wt.%, in particular at 0.011 wt.%, and/or the chromium proportion in the preparation lies between 0.00015 and 0.0017 wt.%, preferably between 0.0002 and 0.0008 wt.%, in particular at 0.00028 wt.%, and/or the selenium proportion in the preparation lies between 0.00015 and 0.0012 wt.%, preferably between 0.0002 and 0.0006 wt.%, in particular at 0.00028 wt.%, and/or the iron proportion in the preparation lies at 0 wt.% or between 0.015 and 0.09 wt.%, preferably between 0.03 and 0.09 wt.%, in particular at 0.08 wt.%, and/or the vitamin D proportion in the preparation lies between 0.00001 and 0.00008 wt.%, preferably between 0.00002 and 0.00006 wt.%, in particular at 0.00004 wt.%, and/or the proportion of catalyst and/or active ingredient for resorption improvement, in particular the piperin proportion, in the preparation lies between 0.0015 and 0.17 wt.%, preferably between 0.005 and 0.034 wt.%, in particular at 0.008 wt.%.

10. Nutritional supplement preparation set according to one of the preceding claims, **characterised in that** in the first and/or the second preparation, in each case the preparation is present in powder form and/or is designed and intended for the production of an aqueous solution and/or is designed and intended for the production of a drink, in particular a mineral-based drink.

## Revendications

1. Complément alimentaire sous forme de kit, comprenant comme substance de base un premier complément alimentaire contenant les sels minéraux et les oligo-éléments qui sont la vitamine D, le calcium, le magnésium, le sélénium, le cuivre, le zinc et le chrome, et au moins un catalyseur et/ou un principe actif qui accroît et/ou améliore et/ou accélère la résorption des sels minéraux et/ou des oligo-éléments, sachant que la première préparation ne contient pas dé fer, et comme additif un deuxième complément alimentaire contenant les sels minéraux et les oligo-éléments qui sont la vitamine D, le calcium, le magnésium, le sélénium, le cuivre, le zinc et le chrome, et au moins un catalyseur et/ou un principe actif qui accroît et/ou améliore et/ou accélère la résorption des sels minéraux et/ou des oligo-éléments, sachant que la deuxième préparation contient du fer,
sachant que la substance de base et l'additif sont miscibles en toutes proportions pour l'ajustement d'une proportion désirée de fer.

2. Complément alimentaire sous forme de kit selon la revendication 1,
**caractérisé en ce que**
dans la première et/ou dans la deuxième préparation respectivement, le au moins un catalyseur et ou principe actif est présent sous forme de poudre.

3. Complément alimentaire sous forme de kit selon la revendication 1 ou 2,
**caractérisé en ce que**
dans la première et/ou la deuxième préparation, le catalyseur et/ou le principe actif respectivement contient un extrait de poivre noir et/ou consiste en cet extrait.

4. Complément alimentaire sous forme de kit selon l'une des revendications précédentes,
**caractérisé en ce que**
dans la première et/ou la deuxième préparation, le catalyseur et/ou le principe actif respectivement contient de la pipérine (désignation chimique: 1-pipéroylpipéridine C₁₇H₁₉NO₃), en particulier comme alcaloïde, et/ou consiste en ce produit, sachant que la pipérine est obtenue de préférence à partir de poivre noir.

5. Complément alimentaire sous forme de kit selon la revendication 4,
**caractérisé en ce que**
dans la première et/ou la deuxième préparation respectivement, la proportion de pipérine dans le catalyseur et/ou le principe actif est comprise dans l'intervalle de 90 % en poids à 100 % en poids, en particulier dans l'intervalle de 92 % en poids à 100 % en poids, de préférence qu'elle est égale à au moins 95 % en poids.

6. Complément alimentaire sous forme de kit selon l'une des revendications précédentes,
**caractérisé en ce que**
dans la première et/ou la deuxième préparation respectivement, le catalyseur et/ou le principe actif reste complètement ou au moins dans une très large mesure conservé dans sa structure moléculaire, telle qu'elle se présente dans le poivre noir.

7. Complément alimentaire sous forme de kit selon l'une des revendications précédentes,
**caractérisé en ce que**
dans la première et/ou la deuxième préparation respectivement, la préparation contient de l'hydroxycarbonate de magnésium.

8. Complément alimentaire sous forme de kit selon l'une des revendications précédentes,
**caractérisé en ce que**
dans la première et/ou la deuxième préparation respectivement, la préparation contient du bêta-carotène (provitamine A) et/ou du potassium et/ou du sodium.

9. Complément alimentaire sous forme de kit selon l'une des revendications précédentes,
**caractérisé en ce que**
dans la première et/ou la deuxième préparation respectivement,
la proportion de potassium dans la préparation est comprise entre 0,15 et 35 % en poids, de préférence entre 0,5 et 10 % en poids, en particulier qu'elle est de 0,8 % en poids, et/ou
la proportion de calcium dans la préparation est comprise entre 1,6 et 17 % en poids, de préférence entre 2,5 et 10 % en poids, en particulier qu'elle est de 4,5 % en poids et/ou
la proportion de magnésium dans la préparation est comprise entre 0,8 et 5,0 % en poids, de préférence entre 2 et 3 % en poids, en particulier qu'elle est de 2,2 % en poids et/ou
la proportion de zinc dans la préparation est comprise entre 0,015 et 0,2 % en poids, de préférence entre 0,05 et 0,1 % en poids, en particulier qu'elle est de 0,08 % en poids et/ou
la proportion de cuivre dans la préparation est comprise entre 0,0015 et 0,017. % en poids, de préférence entre 0,005 et 0,015 % en poids, en particulier qu'elle est de 0,011 % en poids et/ou
la proportion de chrome dans la préparation est comprise entre 0,00015 et 0,0017 % en poids, de préférence entre 0,0002 et 0,0008 % en poids, en particulier qu'elle est de 0,00028 % en poids et/ou
la proportion de sélénium dans la préparation est comprise entre 0,00015 et 0,0012 % en poids, de préférence entre 0,0002 et 0,0006 % en poids, en particulier qu'elle est de 0,00028 % en poids et/ou
la proportion de fer dans la préparation est à peu près de 0 % en poids ou elle est comprise entre 0,015 et 0,09 % en poids, de préférence entre 0,03 et 0,09 % en poids, en particulier qu'elle est de 0,08 % en poids et/ou
la proportion de vitamine D dans la préparation est comprise entre 0,00001 et 0,00008 % en poids, de préférence entre 0,00002 et 0,00006 % en poids, en particulier qu'elle est de 0,00004 % en poids et/ou
la proportion de catalyseur et/ou de principe actif pour améliorer la résorption, en particulier la proportion de pipérine dans la préparation est comprise entre 0,15 et 1,7 % en poids, de préférence entre 0,4 et 1,2 % en poids, en particulier qu'elle est de 0,08 % en poids.

10. Complément alimentaire sous forme de kit selon l'une des revendications précédentes,
**caractérisé en ce que**
dans la première et/ou la deuxième préparation respectivement, la préparation se présente sous forme de poudre et/ou qu'elle est configurée et destinée à la préparation d'une solution aqueuse et/ou qu'elle est configurée et destinée à la préparation d'une boisson, en particulier d'une boisson minérale basique.
